# EUROPEAN PATENT APPLICATION

(11) **EP 1 693 070 A1**
(43) Date of publication of application: **23.08.2006**
(21) Application number: 04819443.5
(22) Date of filing: 26.11.2004
(51) Int. Cl.: A61K 45/00, A61P 1/00, A61P 1/04, A61P 29/00, A61P 31/04, C12N 15/57, C12N 15/12

(54) **PROCASPASE 1 ACTIVATION INHIBITOR**

(30) Priority: 26.11.2003 JP 2003396278
(71) Applicant: DAIICHI PHARMACEUTICAL CO., LTD., Chuo-ku, Tokyo 103-8234 (JP)
(72) Inventor: DOI, Hirofumi Celestar Lexico-Sciences, Inc., Chiba-shi, Chiba 261-8501 (JP); MASUDA, Shoichi Celestar Lexico-Sciences, Inc., Chiba-shi, Chiba 261-8501 (JP); ISUMI, Yoshitaka Daiichi Pharmaceutical Co., Ltd., Edogawa-ku, Tokyo 134-8630 (JP)
(74) Representative: Zwicker, Jörk
(86) International application number: PCT/JP2004/017586
(87) International publication number: WO 2005/051425

(57) **Abstract**

A method and an agent for inhibiting the oligomerization of procaspase-1, a method and an agent for inhibiting the activation of procaspase-1, a method and an agent for inhibiting the production of caspase-1, a method of preventing and/or treating an inflammatory disease, and an agent for preventing and/or treating an inflammatory disease, all of which comprise inhibiting the binding of NOD2 to procaspase-1; a method of identifying a compound that inhibits the binding of NOD2 to procaspase-1; and a reagent kit for use in the identifying method are provided.

SEQUENCE LISTING

<110>  DAIICHI PHARMACEUTICAL CO., LTD.

<120>  An inhibitor of procaspase-1 activation

<130>  535-2

<150>  P2003-396278
<151>  2003-11-26

<160>  4

<170>  PatentIn version 3.1

<210>  1
<211>  3120
<212>  DNA
<213>  Homo sapiens

<220>
<221>  CDS
<222>  (1)..(3120)
<223>

<400>  1
atg ggg gaa gag ggt ggt tca gcc tct cac gat gag gag gaa aga gca      48
agt gtc ctc ctc gga cat tct ccg ggt tgt gaa atg tgc tcg cag gag      96
gct ttt cag gca cag agg agc cag ctg gtc gag ctg ctg gtc tca ggg      144
tcc ctg gaa ggc ttc gag agt gtc ctg gac tgg ctg ctg tcc tgg gag      192
gtc ctc tcc tgg gag gac tac gag ggc ttc cac ctc ctg ggc cag cct      240
ctc tcc cac ttg gcc agg cgc ctt ctg gac acc gtc tgg aat aag ggt      288
act tgg gcc tgt cag aag ctc atc gcg gct gcc caa gaa gcc cag gcc      336
gac agc cag tcc ccc aag ctg cat ggc tgc tgg gac ccc cac tcg ctc      384
cac cca gcc cga gac ctg cag agt cac cgg cca gcc att gtc agg agg      432
ctc cac agc cat gtg gag aac atg ctg gac ctg gca tgg gag cgg ggt      480
ttc gtc agc cag tat gaa tgt gat gaa atc agg ttg ccg atc ttc aca      528
ccg tcc cag agg gca aga agg ctg ctt gat ctt gcc acg gtg aaa gcg      576
aat gga ttg gct gcc ttc ctt cta caa cat gtt cag gaa tta cca gtc      624
cca ttg gcc ctg cct ttg gaa gct gcc aca tgc aag aag tat atg gcc      672
aag ctg agg acc acg gtg tct gct cag tct cgc ttc ctc agt acc tat      720
gat gga gca gag acg ctc tgc ctg gag gac ata tac aca gag aat gtc      768
ctg gag gtc tgg gca gat gtg ggc atg gct gga ccc ccg cag aag agc      816
cca gcc acc ctg ggc ctg gag gag ctc ttc agc acc cct ggc cac ctc      864
aat gac gat gcg gac act gtg ctg gtg gtg ggt gag gcg ggc agt ggc      912
aag agc acg ctc ctg cag cgg ctg cac ttg ctg tgg gct gca ggg caa      960
gac ttc cag gaa ttt ctc ttt gtc ttc cca ttc agc tgc cgg cag ctg     1008
cag tgc atg gcc aaa cca ctc tct gtg cgg act cta ctc ttt gag cac     1056
tgc tgt tgg cct gat gtt ggt caa gaa gac atc ttc cag tta ctc ctt     1104
gac cac cct gac cgt gtc ctg tta acc ttt gat ggc ttt gac gag ttc     1152
aag ttc agg ttc acg gat cgt gaa cgc cac tgc tcc ccg acc gac ccc     1200
acc tct gtc cag acc ctg ctc ttc aac ctt ctg cag ggc aac ctg ctg 1248
aag aat gcc cgc aag gtg gtg acc agc cgt ccg gcc gct gtg tcg gcg 1296
ttc ctc agg aag tac atc cgc acc gag ttc aac ctc aag ggc ttc tct 1344
gaa cag ggc atc gag ctg tac ctg agg aag cgc cat cat gag ccc ggg 1392
gtg gcg gac cgc ctc atc cgc ctg ctc caa gag acc tca gcc ctg cac 1440
ggt ttg tgc cac ctg cct gtc ttc tca tgg atg gtg tcc aaa tgc cac 1488
cag gaa ctg ttg ctg cag gag ggg ggg tcc cca aag acc act aca gat 1536
atg tac ctg ctg att ctg cag cat ttt ctg ctg cat gcc acc ccc cca 1584
gac tca gct tcc caa ggt ctg gga ccc agt ctt ctt cgg ggc cgc ctc 1632
ccc acc ctc ctg cac ctg ggc aga ctg gct ctg tgg ggc ctg ggc atg 1680
tgc tgc tac gtg ttc tca gcc cag cag ctc cag gca gca cag gtc agc 1728
cct gat gac att tct ctt ggc ttc ctg gtg cgt gcc aaa ggt gtc gtg 1776
cca ggg agt acg gcg ccc ctg gaa ttc ctt cac atc act ttc cag tgc 1824
ttc ttt gcc gcg ttc tac ctg gca ctc agt gct gat gtg cca cca gct 1872
ttg ctc aga cac ctc ttc aat tgt ggc agg cca ggc aac tca cca atg 1920
gcc agg ctc ctg ccc acg atg tgc atc cag gcc tcg gag gga aag gac 1968
agc agc gtg gca gct ttg ctg cag aag gcc gag ccg cac aac ctt cag     2016
atc aca gca gcc ttc ctg gca ggg ctg ttg tcc cgg gag cac tgg ggc     2064
ctg ctg gct gag tgc cag aca tct gag aag gcc ctg ctc cgg cgc cag     2112
gcc tgt gcc cgc tgg tgt ctg gcc cgc agc ctc cgc aag cac ttc cac     2160
tcc atc ccg cca gct gca ccg ggt gag gcc aag agc gtg cat gcc atg     2208
ccc ggg ttc atc tgg ctc atc cgg agc ctg tac gag atg cag gag gag     2256
cgg ctg gct cgg aag gct gca cgt ggc ctg aat gtt ggg cac ctc aag     2304
ttg aca ttt tgc agt gtg ggc ccc act gag tgt gct gcc ctg gcc ttt     2352
gtg ctg cag cac ctc cgg cgg ccc gtg gcc ctg cag ctg gac tac aac     2400
tct gtg ggt gac att ggc gtg gag cag ctg ctg cct tgc ctt ggt gtc     2448
tgc aag gct ctg tat ttg cgc gat aac aat atc tca gac cga ggc atc     2496
tgc aag ctc att gaa tgt gct ctt cac tgc gag caa ttg cag aag tta     2544
gct cta ttc aac aac aaa ttg act gac ggc tgt gca cac tcc atg gct     2592
aag ctc ctt gca tgc agg cag aac ttc ttg gca ttg agg ctg ggg aat     2640
aac tac atc act gcc gcg gga gcc caa gtg ctg gcc gag ggg ctc cga     2688
ggc aac acc tcc ttg cag ttc ctg gga ttc tgg ggc aac aga gtg ggt     2736
gac gag ggg gcc cag gcc ctg gct gaa gcc ttg ggt gat cac cag agc      2784
ttg agg tgg ctc agc ctg gtg ggg aac aac att ggc agt gtg ggt gcc      2832
caa gcc ttg gca ctg atg ctg gca aag aac gtc atg cta gaa gaa ctc      2880
tgc ctg gag gag aac cat ctc cag gat gaa ggt gta tgt tct ctc gca      2928
gaa gga ctg aag aaa aat tca agt ttg aaa atc ctg aag ttg tcc aat      2976
aac tgc atc acc tac cta ggg gca  gaa gcc ctc ctg cag  gcc ctt gaa    3024
agg aat  gac acc atc ctg gaa  gtc tgg ctc cga ggg  aac act ttc       3069
tct cta  gag gag gtt gac aag  ctc ggc tgc agg gac  acc aga ctc       3114
ttg ctt                                                              3120


<210>  2
<211>  1040
<212>  PRT
<213>  Homo sapiens

<400>  2

<210>  3
<211>  1212
<212>  DNA
<213>  Homo sapiens

<220>
<221> CDS
<222> (1)..(1212)
<223>

<400> 3
atg gcc gac aag gtc ctg aag gag aag aga aag ctg ttt atc cgt tcc 48
Met Ala Asp Lys Val Leu Lys Glu Lys Arg Lys Leu Phe Ile Arg Ser
1               5                   10                  15

atg ggt gaa ggt aca ata aat ggc tta ctg gat gaa tta tta cag aca 96
Met Gly Glu Gly Thr Ile Asn Gly Leu Leu Asp Glu Leu Leu Gln Thr
            20                  25                  30

agg gtg ctg aac aag gaa gag atg gag aaa gta aaa cgt gaa aat gct 144
Arg Val Leu Asn Lys Glu Glu Met Glu Lys Val Lys Arg Glu Asn Ala
        35                  40                  45

aca gtt atg gat aag acc cga gct ttg att gac tcc gtt att ccg aaa 192
Thr Val Met Asp Lys Thr Arg Ala Leu Ile Asp Ser Val Ile Pro Lys
    50                  55                  60

ggg gca cag gca tgc caa att tgc atc aca tac att tgt gaa gaa gac 240
Gly Ala Gln Ala Cys Gln Ile Cys Ile Thr Tyr Ile Cys Glu Glu Asp
65                  70                  75                  80

agt tac ctg gca ggg acg ctg gga ctc tca gca gat caa aca tct gga 288
Ser Tyr Leu Ala Gly Thr Leu Gly Leu Ser Ala Asp Gln Thr Ser Gly
                85                  90                  95

aat tac ctt aat atg caa gac tct caa gga gta ctt tct tcc ttt cca 336
Asn Tyr Leu Asn Met Gln Asp Ser Gln Gly Val Leu Ser Ser Phe Pro
            100                 105                 110

gct cct cag gca gtg cag gac aac cca gct atg ccc aca tcc tca ggc 384
Ala Pro Gln Ala Val Gln Asp Asn Pro Ala Met Pro Thr Ser Ser Gly
        115                 120                 125

tca gaa ggg aat gtc aag ctt tgc tcc cta gaa gaa gct caa agg ata 432
Ser Glu Gly Asn Val Lys Leu Cys Ser Leu Glu Glu Ala Gln Arg Ile
    130                 135                 140

tgg aaa caa aag tcg gca gag att tat cca ata atg gac aag tca agc 480
Trp Lys Gln Lys Ser Ala Glu Ile Tyr Pro Ile Met Asp Lys Ser Ser
145                 150                 155                 160

cgc aca cgt ctt gct ctc att atc tgc aat gaa gaa ttt gac agt att 528
Arg Thr Arg Leu Ala Leu Ile Ile Cys Asn Glu Glu Phe Asp Ser Ile
                165                 170                 175

cct aga aga act gga gct gag gtt gac atc aca ggc atg aca atg ctg 576
Pro Arg Arg Thr Gly Ala Glu Val Asp Ile Thr Gly Met Thr Met Leu
            180                 185                 190

cta caa aat ctg ggg tac agc gta gat gtg aaa aaa aat ctc act gct 624
Leu Gln Asn Leu Gly Tyr Ser Val Asp Val Lys Lys Asn Leu Thr Ala
        195                 200                 205

tcg gac atg act aca gag ctg gag gca ttt gca cac cgc cca gag cac 672
Ser Asp Met Thr Thr Glu Leu Glu Ala Phe Ala His Arg Pro Glu His
    210                 215                 220

aag acc tct gac agc acg ttc ctg gtg ttc atg tct cat ggt att cgg 720

Lys Thr Ser Asp Ser Thr Phe Leu Val Phe Met Ser His Gly Ile Arg
225                 230                 235                 240

gaa ggc att tgt ggg aag aaa cac tct gag caa gtc cca gat ata cta      768
Glu Gly Ile Cys Gly Lys Lys His Ser Glu Gln Val Pro Asp Ile Leu
                245                 250                 255

caa ctc aat gca atc ttt aac atg ttg aat acc aag aac tgc cca agt      816
Gln Leu Asn Ala Ile Phe Asn Met Leu Asn Thr Lys Asn Cys Pro Ser
            260                 265                 270

ttg aag gac aaa ccg aag gtg atc atc atc cag gcc tgc cgt ggt gac      864
Leu Lys Asp Lys Pro Lys Val Ile Ile Ile Gln Ala Cys Arg Gly Asp
        275                 280                 285

agc cct ggt gtg gtg tgg ttt aaa gat tca gta gga gtt tct gga aac      912
Ser Pro Gly Val Val Trp Phe Lys Asp Ser Val Gly Val Ser Gly Asn
    290                 295                 300

cta tct tta cca act aca gaa gag ttt gag gat gat gct att aag aaa      960
Leu Ser Leu Pro Thr Thr Glu Glu Phe Glu Asp Asp Ala Ile Lys Lys
305                 310                 315                 320

gcc cac ata gag aag gat ttt atc gct ttc tgc tct tcc aca cca gat     1008
Ala His Ile Glu Lys Asp Phe Ile Ala Phe Cys Ser Ser Thr Pro Asp
                325                 330                 335

aat gtt tct tgg aga cat ccc aca atg ggc tct gtt ttt att gga aga     1056
Asn Val Ser Trp Arg His Pro Thr Met Gly Ser Val Phe Ile Gly Arg
            340                 345                 350

ctc att gaa cat atg caa gaa tat gcc tgt tcc tgt gat gtg gag gaa     1104
Leu Ile Glu His Met Gln Glu Tyr Ala Cys Ser Cys Asp Val Glu Glu
        355                 360                 365

att ttc cgc aag gtt cga ttt tca ttt gag cag cca gat ggt aga gcg     1152
Ile Phe Arg Lys Val Arg Phe Ser Phe Glu Gln Pro Asp Gly Arg Ala
    370                 375                 380

cag atg ccc acc act gaa aga gtg act ttg aca aga tgt ttc tac ctc     1200
Gln Met Pro Thr Thr Glu Arg Val Thr Leu Thr Arg Cys Phe Tyr Leu
385                 390                 395                 400

ttc cca gga cat                                                     1212
Phe Pro Gly His

<210>  4
<211>  404
<212>  PRT
<213>  Homo sapiens

<400>  4

Met Ala Asp Lys Val Leu Lys Glu Lys Arg Lys Leu Phe Ile Arg Ser
1               5                   10                  15

Met Gly Glu Gly Thr Ile Asn Gly Leu Leu Asp Glu Leu Leu Gln Thr
            20                  25                  30

Arg Val Leu Asn Lys Glu Glu Met Glu Lys Val Lys Arg Glu Asn Ala
        35                  40                  45

Thr Val Met Asp Lys Thr Arg Ala Leu Ile Asp Ser Val Ile Pro Lys
    50                  55                  60

Gly Ala Gln Ala Cys Gln Ile Cys Ile Thr Tyr Ile Cys Glu Glu Asp
65                  70                  75                  80

Ser Tyr Leu Ala Gly Thr Leu Gly Leu Ser Ala Asp Gln Thr Ser Gly
                85                  90                  95

Asn Tyr Leu Asn Met Gln Asp Ser Gln Gly Val Leu Ser Ser Phe Pro
            100                 105                 110

Ala Pro Gln Ala Val Gln Asp Asn Pro Ala Met Pro Thr Ser Ser Gly
        115                 120                 125

Ser Glu Gly Asn Val Lys Leu Cys Ser Leu Glu Glu Ala Gln Arg Ile
    130                 135                 140

Trp Lys Gln Lys Ser Ala Glu Ile Tyr Pro Ile Met Asp Lys Ser Ser
145                 150                 155                 160

Arg Thr Arg Leu Ala Leu Ile Ile Cys Asn Glu Glu Phe Asp Ser Ile
                165                 170                 175

Pro Arg Arg Thr Gly Ala Glu Val Asp Ile Thr Gly Met Thr Met Leu
            180                 185                 190

Leu Gln Asn Leu Gly Tyr Ser Val Asp Val Lys Lys Asn Leu Thr Ala
        195                 200                 205

Ser Asp Met Thr Thr Glu Leu Glu Ala Phe Ala His Arg Pro Glu His
    210                 215                 220

Lys Thr Ser Asp Ser Thr Phe Leu Val Phe Met Ser His Gly Ile Arg
225                 230                 235                 240

Glu Gly Ile Cys Gly Lys Lys His Ser Glu Gln Val Pro Asp Ile Leu
                245                 250                 255

Gln Leu Asn Ala Ile Phe Asn Met Leu Asn Thr Lys Asn Cys Pro Ser
            260                 265                 270

Leu Lys Asp Lys Pro Lys Val Ile Ile Ile Gln Ala Cys Arg Gly Asp
        275                 280                 285

Ser Pro Gly Val Val Trp Phe Lys Asp Ser Val Gly Val Ser Gly Asn
    290                 295                 300

Leu Ser Leu Pro Thr Thr Glu Glu Phe Glu Asp Asp Ala Ile Lys Lys
305                 310                 315                 320

Ala His Ile Glu Lys Asp Phe Ile Ala Phe Cys Ser Ser Thr Pro Asp
                325                 330                 335

Asn Val Ser Trp Arg His Pro Thr Met Gly Ser Val Phe Ile Gly Arg
            340                 345                 350

Leu Ile Glu His Met Gln Glu Tyr Ala Cys Ser Cys Asp Val Glu Glu
        355                 360                 365

Ile Phe Arg Lys Val Arg Phe Ser Phe Glu Gln Pro Asp Gly Arg Ala
    370                 375                 380

Gln Met Pro Thr Thr Glu Arg Val Thr Leu Thr Arg Cys Phe Tyr Leu
385                 390                 395                 400

Phe Pro Gly His

## Description

### TECHNICAL FIELD

The present invention relates to a method of inhibiting the oligomerization of procaspase-1, a method of inhibiting the activation of procaspase-1, and a method of inhibiting the production of caspase-1, comprising inhibiting the binding of NOD2 to procaspase-1. Further, it relates to a method of preventing and/or treating an inflammatory disease, comprising inhibiting the binding of NOD2 to procaspase-1. Further, it also relates to a method of identifying a compound which inhibits a binding of NOD2 to procaspase-1. Further, it also relates to an agent for inhibiting the oligomerization of procaspase-1, an agent for inhibiting the activation of procaspase-1, and an agent for inhibiting the production of caspase-1, or an agent for preventing and/or treating an inflammatory disease, which inhibits the binding of NOD2 to procaspase-1. Further, it relates to a reagent kit comprising at least one member selected from NOD2, a polynucleotide encoding NOD2, a vector comprising the polynucleotide, and a transformant comprising the vector, and at least one member selected from procaspase-1, a polynucleotide encoding procaspase-1, a vector comprising the polynucleotide, and a transformant comprising the vector.

### BACKGROUND ART

Caspase-1, which is also referred to as interleukin-1β-converting enzyme (ICE), is a cysteine protease which converts the precursors of inflammatory cytokines, interleukin 1β (hereinafter, abbreviated as IL-1β) and interleukin 18 (hereinafter, abbreviated as IL-18), into mature form (Non-patent Documents 1-4). Caspase-1 gene is induced by an inflammatory stimuli such as a lipopolysaccharide (hereinafter, abbreviated as LPS), and likewise caspase-1 activity is increased by a similar stimuli (Non-patent Documents 4-6). Further, it has been reported that caspase-1 activity is enhanced in various inflammatory diseases, for example, sepsis, inflammatory bowel disease, and rheumatic disorder.

It is postulated that caspase-1 is produced from procaspase-1, a protein being expressed as a caspase-1 precursor, and by oligomerization thereof followed by its autocleavage. It has been reported that the oligomerization of procaspase-1 in that process is induced by the binding of RIP2 or NOD1 to procaspase-1 (Non-patent Documents 7 and 8).

The literatures cited herein are listed below.
Patent Document 1: International Publication No. WO 01/67299
Non-patent Document 1: Nature, 1992, 356: 768-774.
Non-patent Document 2: Science, 1992, 256: 97-100.
Non-patent Document 3: Nature, 1997, 386: 619-623.
Non-patent Document 4: Science, 1997, 275: 206-209.
Non-patent Document 5: Nature, 1994, 370: 270-275.
Non-patent Document 6: Blood, 1998, 91: 577-584.
Non-patent Document 7: Current Biology, 1998, 8: 885-888.
Non-patent Document 8: Biochemical and Biophysical Research Communications, 2002, 299: 652-658.
Non-patent Document 9: Journal of Biological Chemistry, 2002, 277: 41701-41705.
Non-patent Document 10: Gut, 2003, 52: 840-846.
Non-patent Document 11: Journal of Biological Chemistry, 2001, 276: 4812-4818.
Non-patent Document 12: K. M. Ulmer, Science, 1983, 219: 666-671.
Non-patent Document 13: Peptide Synthesis, Maruzen Co. Ltd., 1975.
Non-patent Document 14: Peptide Synthesis, Interscience, New York, 1996.
Non-patent Document 15: Cell, 1995, 80: 401-411.
Non-patent Document 16: Science, 1995, 267: 2000-2003.
Non-patent Document 17: Proceedings of the National Academy of Sciences of the United States of America, 2001, 98: 13249-13254.
Non-patent Document 18: Nature, 2003, 423: 356-361.
Non-patent Document 19: Biochemical Pharmacology, 2002, 64: 1-8.

### DISCLOSURE OF THE INVENTION

### (PROBLEMS TO BE SOLVED BY THE INVENTION)

An object of the present invention is to obtain a novel protein which binds to procaspase-1 to facilitate the oligomerization and activation thereof, and then to provide a means comprising inhibiting the binding of the protein to procaspase-1 for allowing for the inhibition of oligomerization of procaspase-1, the inhibition of activation of procaspase-1 and the inhibition of production of caspase-1 as well as the prevention and/or treatment of an inflammatory disease.

### (MEANS FOR SOLVING THE PROBLEM)

The present inventors have concentrated their efforts to meet the aforementioned object, and predicted *in-silico* that procaspase-1 interacts with NOD2. Then, the present inventors proved experimentally that NOD2 binds to procaspase-1, thereby to facilitate the oligomerization of procaspase-1, which resulted in induction of the activation of procaspase-1 to caspase-1 and the production of caspase-1. The present invention has been thus achieved.

In various embodiments, the present invention relates to a method selected from the following group:
(i) a method of inhibiting the oligomerization of procaspase-1, comprising inhibiting the binding ofNOD2 to procaspase-1,
(ii) a method of inhibiting the activation of procaspase-1, comprising inhibiting the binding of NOD2 to procaspase-1, and
(iii) a method of inhibiting the production of caspase-1, comprising inhibiting the binding of NOD2 to procaspase-1.

The present invention further relates to a method of preventing and/or treating an inflammatory disease, comprising inhibiting the binding ofNOD2 to procaspase-1.

The present invention still further relates to a method of preventing and/or treating an inflammatory disease, comprising using at least one compound that inhibits the binding of NOD2 to procaspase-1.

The present invention also relates to the aforementioned method of preventing and/or treating an inflammatory disease, wherein the inflammatory disease is sepsis, inflammatory bowel disease, Crohn's disease or rheumatic disorder.

The present invention further relates to a method of identifying a compound that inhibits the binding of NOD2 to procaspase-1, comprising contacting NOD2 and/or procaspase-1 with a compound under conditions that allow for the binding of NOD2 to procaspase-1, employing a system using a signal and/or marker capable of the detecting the binding of NOD2 to procaspase-1; and detecting the presence or absence and/or change of the signal and/or marker to determine whether the compound inhibits the binding ofNOD2 to procaspase-1.

The present invention still further relates to an agent selected from the following group:
(i) an agent for inhibiting the oligomerization of procaspase-1, which inhibits the binding of NOD2 to procaspase-1,
(ii) an agent for inhibiting the activation of procaspase-1, which inhibits the binding of NOD2 to procaspase-1, and
(iii) an agent for inhibiting the production of caspase-1, which inhibits the binding of NOD2 to procaspase-1.

The present invention also relates to an agent selected from the following groups:
(i) an agent for inhibiting the oligomerization of procaspase-1, comprising at least one compound that inhibits the binding ofNOD2 to procaspase-1,
(ii) an agent for inhibiting the activation of procaspase-1, comprising at least one compound that inhibits the binding ofNOD2 to procaspase-1, and
(iii) an agent for inhibiting the production of caspase-1, comprising at least one compound that inhibits the binding ofNOD2 to procaspase-1.

The present invention further relates to an agent for preventing and/or treating an inflammatory disease, which inhibits the binding ofNOD2 to procaspase-1.

The present invention still further relates to an agent for preventing and/or treating an inflammatory disease, comprising at least one compound that inhibits the binding of NOD2 to procaspase-1.

The present invention also relates to an agent for preventing and/or treating an inflammatory disease, comprising the aforementioned inhibitory agent.

The present invention further relates to any one of the aforementioned agents for preventing and/or treating an inflammatory disease, wherein the inflammatory disease is sepsis, inflammatory bowel disease, Crohn's disease, or rheumatic disorder.

The present invention still further relates to an agent for preventing and/or treating an inflammatory disease, which inhibits the oligomerization of procaspase-1 by inhibiting the binding ofNOD2 to procaspase-1.

The present invention also relates to any one of the aforementioned agents for preventing and/or treating an inflammatory disease, wherein the inflammatory disease is sepsis, inflammatory bowel disease, Crohn's disease, or rheumatic disorder.

The present invention further relates to a reagent kit for use in the aforementioned identification method, comprising at least one selected from NOD2, a polynucleotide encoding NOD2, a vector comprising the polynucleotide and a transformant comprising the vector; and at least one selected from procaspase-1, a polynucleotide encoding procaspase-1, a vector comprising the polynucleotide and a transformant comprising the vector.

### ADVANTAGE OF THE INVENTION

In the present invention, it was discovered that NOD2 binds to procaspase-1. Namely, it was proved for the first time that NOD2 binds to procaspase-1, thereby to facilitate the oligomerization of procaspase-1, which resulted in the induction of the activation of procaspase-1 to caspase-1 and the production of caspase-1. Caspase-1 is a factor involved in inflammatory reactions, and it has been reported that its activity is enhanced in an inflammatory disease. Thus, the present invention comprising inhibiting the binding of NOD2 to procaspase-1 is quite useful for preventing and/or treating an inflammatory disease.

### BRIEF DESCRIPTION OF THE DRAWINGS

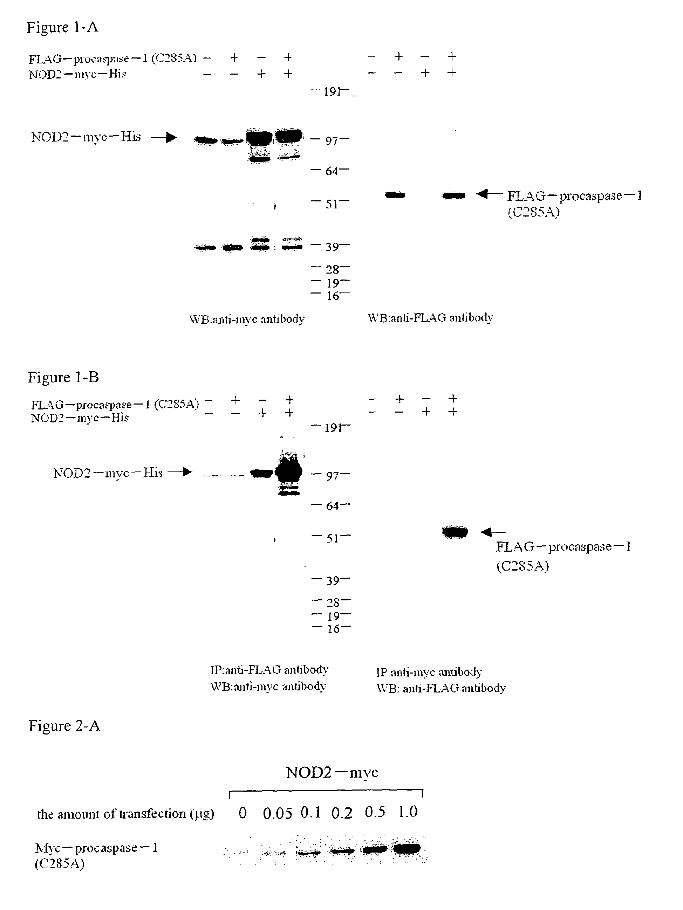

Fig. 1-A shows the amount of protein, which was expressed by transfecting a cell with a FLAG-procaspase-1 (C285A) expression plasmid alone, an NOD2-myc-His expression plasmid alone, or the both plasmids in combination, in a cell lysate prepared from the cell. The protein was detected by Western blotting (WB). (Example 2)
Fig. 1-B shows in the left panel that a cell lysate prepared from a cell that was made to co-express NOD2-myc-His with FLAG-procaspase-1 (C285A) was subjected to immunoprecipitation (IP) using anti-FLAG M2 antibody, and as a result, a band of NOD2-myc-His was detected. The right panel shows that the aforementioned cell lysate was subjected to immunoprecipitation using anti-myc antibody, and as a result, the band of FLAG-procaspase-1 (C285A) was detected only in a lysate of a cell that was made to co-express NOD2-myc-His with FLAG-procaspase-1 (C285A). The proteins were detected by Western blotting (WB). (Example 2)

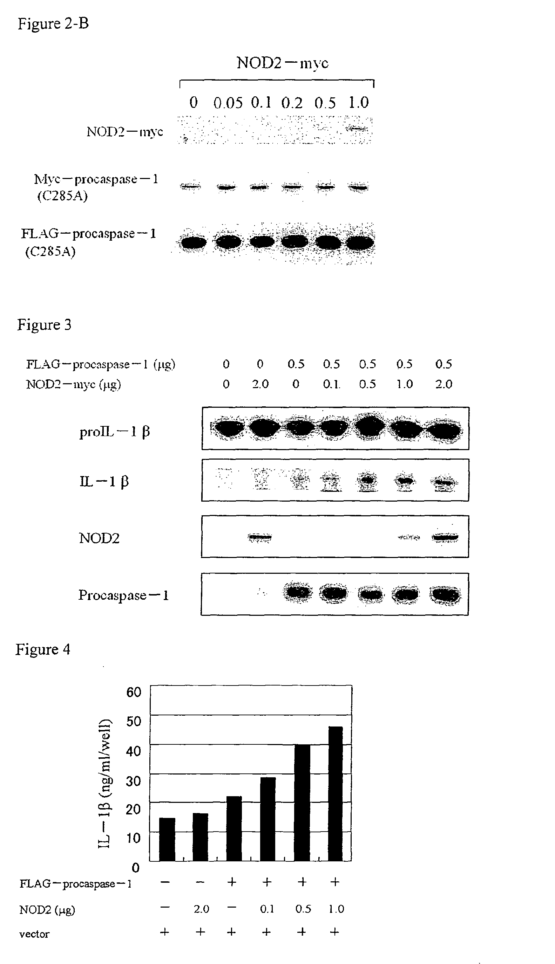

Figure 2-A demonstrates that a cell lysate prepared from a cell transfected with FLAG-procaspase-1 (C285A) expression plasmid, Myc-procaspase-1 (C285A) expression plasmid and NOD2-myc expression plasmid at indicated concentrations was treated with anti-FLAG antibody to give a fraction, in which the amount of the band detected with an anti-myc antibody increased in a manner dependent on the amount of NOD2-myc expression plasmid. (Example 3)
Figure 2-B shows the amount of respective proteins, which were expressed by transfecting a cell with FLAG-procaspase-1 (C285A) expression plasmid, Myc-procaspase-1 (C285A) expression plasmid and NOD2-myc expression plasmid at indicated concentrations, in a cell lysate prepared from the cell. (Example 3)
Figure 3 demonstrates that a cell which was made to co-express FLAG-procaspase-1 expression plasmid with NOD2-myc expression plasmid showed an increase in the amount of intracellular IL-1β in a manner dependent on the amount of expressed NOD2-myc. (Example 4)
Figure 4 demonstrates that a cell which was made to co-express FLAG-procaspase-1 expression plasmid (0.5 µg) with NOD2 expression plasmid showed an increase in the amount of IL-1β secreted outside the cell in a manner dependent on the amount of NOD2 expression plasmid. In the figure, the term "vector" means an empty vector which was added to adjust the amount of DNA introduced to each cell to be the same. (Example 4)

### DETAILED DESCRIPTION OF THE INVENTION

Embodiments of the present invention are explained in further detail below.
In the present specification, the term "polypeptide" may be used as a generic term which includes the following: an isolated or a synthetic full-length protein; an isolated or a synthetic full-length polypeptide; and an isolated or a synthetic full-length oligopeptide. A protein, a polypeptide or an oligopeptide used herein comprises two or more amino acids that are bound to each other by a peptide bond or a modified peptide bond. Hereinafter, an amino acid may be represented by a single letter or by three letters.

In the present invention, the interaction of NOD2 with procaspase-1 was predicted *in-silico* according to the method described in the International Publication No. WO 01/67299. Then, it has been demonstrated experimentally that NOD2 binds to procaspase-1. Furthermore, it has been clarified for the first time that NOD2 binds bound to procaspase-1, and thereby to facilitate the oligomerization of procaspase-1, resulting in induction of the activation of procaspase-1 to caspase-1 and the production of caspase-1, and thereby to induce caspase-dependent cleavage of IL-1β precursor (pro IL-1β) in a cell to facilitate the secretion of mature IL-1β.

NOD2 is also called as CARD15, and is reported to be expressed in a monocyte (Non-patent Document 9), neutrophil (Non-patent Document 9), leukocyte (Non-patent Document 9), granulocyte (Non-patent Document 9), dendritic cell (Non-patent Document 9), intestinal epithelium cell (Non-patent Documents 9 and 10) and macrophage (Non-patent Document 10). In addition, a NOD2 gene is reported to increase by an inflammatory stimuli, such as LPS and tumor necrosis factor α (hereinafter, abbreviated as TNF-α) in an acute promyelocytic leukemia cell strain, HL-60, and a normal colon cell strain FHC, (Non-patent Document 9). Concerning a function of NOD2, it is reported that the transient expression of NOD2 gene in HL-60 cell leads to activation of NF-κB (Non-patent Document 11). However, there is no report demonstrating that NOD2 binds to procaspase-1 to exhibit the function.

Therefore, to inhibit the binding of NOD2 to procaspase-1 allows inhibition of the oligomerization of procaspase-1, inhibition of the activation of procaspase-1 and inhibition of the production of caspase-1, which results in allowing for the prevention and/or treatment of an inflammatory disease which occurs and progresses due to the increase of caspase-1.

Based on these findings, the present invention provides a method of inhibiting the oligomerization of procaspase-1, a method of inhibiting the activation of procaspase-1 and a method of inhibiting the production of caspase-1, all of which comprises inhibiting the binding of NOD2 to procaspase-1, as well as a method of preventing and/or treating a disease attributable to the increase of caspase-1, in particular an inflammatory disease.

A NOD2 gene and a polypeptide encoded by the gene comprise sequences shown in SEQ ID NOs: 1 and 2 in the sequence listing, respectively. A procaspase-1 gene and a polypeptide encoded by the gene comprise sequences shown in SEQ ID NOs: 3 and 4 in the sequence listing, respectively. NOD2, procaspase-1 and genes thereof are not limited to those comprising the respective sequences described above, and they may be a polypeptide and a polynucleotide which have one to several mutations in the respective sequences described above insofar as they have the functions of NOD2 and procaspase-1 which are known in general. Further, they may be a variant in which one to several mutations has been introduced in the respective sequences described above for purposes of a desired change such as facilitation or elimination of the functions thereof.

The phrase "the binding of NOD2 to procaspase-1" refers to the interaction of NOD2 with procaspase-1 so as to form a complex by a non-covalent bond such as a hydrogen bond, a hydrophobic bond, an electrically static interaction or the like. The binding of NOD2 to procaspase-1 only at the portion of these molecules is enough to be referred as "the binding" mentioned herein. For example, an amino acid which is not involved in the binding of NOD2 to procaspase-1 may be contained in the amino acid that constitutes NOD2 or procaspase-1.

The binding of NOD2 to procaspase-1 can be detected by a method well known in the art, such as an immunoprecipitation method for confirming co-precipitated product, a two-hybrid method, a Western blotting, a fluorescence resonance energy transfer method and the like, or by using these methods in combination.

For example, as shown in Example 2, the binding of C-terminal myc-His-tagged NOD2 to N-terminal FLAG-tagged procaspase-1 can be detected by conducting immunoprecipitation under co-existence of the both polypeptides using anti-myc antibody or anti-FLAG M2 antibody to give a coprecipitate followed by subjecting the coprecipitate to Western blotting using anti-FLAG M2 antibody or anti-myc antibody.

The phrase "the oligomerization of procaspase-1 " means that procaspase-1 binds to each other, thereby to form a complex. The oligomerization is facilitated by the binding of NOD2 to procaspase-1. Further, procaspase-1 contains the caspase recruitment domain (CARD) that is known to play a key role in oligomerization thereof. The number of molecules of procaspase-1 constituting the complex is not limited in particular, and it can be a number such that procaspase-1 binds to each other, thereby to form a complex to the extent allowing procaspase-1 to cleave itself which results in the production ofcaspase-1.

The phrase "the activation of procaspase-1" means that procaspase-1 is autocleaved along with the oligomerization of procaspase-1 to produce caspase-1. The binding of NOD2 to procaspase-1 facilitates the oligomerization of procaspase-1 followed by induction of autocleavage thereof, resulting in the production of caspase-1.

The phrase "the production of caspase-1" means that caspase-1 is produced as a result of the activation of procaspase-1.

The inhibition of the oligomerization of procaspase-1, the inhibition of the activation of procaspase-1, and the inhibition of the production of caspase-1 can be achieved by, for example, using a compound capable of inhibiting the binding of NOD2 to procaspase-1. As used herein, a compound having such an inhibitory effect (as the example described later, polypeptides having a competitive inhibiting effect, antibody and compound having a lower molecular weight and the like are listed) is referred to as an inhibiting agent.

A compound that inhibits the binding of NOD2 to procaspase-1 is preferably a compound that specifically inhibits the binding, and more preferably, a compound with a lower molecular weight which specifically inhibits the binding. "Specifically inhibit the binding of NOD2 to procaspase-1" denotes inhibiting that binding strongly, but does not inhibit or weakly inhibit the binding between the other proteins.

As a compound that inhibits the binding of NOD2 to procaspase-1, a polypeptide comprising the amino acid sequence of a site where NOD2 binds to procaspase-1 can be exemplified. Such a polypeptide can competitively inhibit the protein-protein binding, and thereby inhibit the oligomerization of procaspase-1 and the autocleavage of the same. Such a polypeptide can be obtained by designing polypeptides based on the amino acid sequence of NOD2 or procaspase-1, synthesizing them by peptide synthesis methods well-known in the art, and selecting a polypeptide that inhibits the binding of NOD2 to procaspase-1 from them. A polypeptide having an amino acid sequence derived from the thus specified polypeptide, in which a mutation such as a deletion, substitution, addition or insertion of one to several amino acids was introduced, is also included in the scope of the present invention. Among the polypeptides into which such a mutation has been introduced, a polypeptide inhibiting the binding of NOD2 to procaspase-1 is preferably used. A polypeptide having the mutation may be a naturally existing polypeptide or a polypeptide in which a mutation was introduced. Techniques for introducing a mutation such as a deletion, substitution, addition or insertion are known. For example, the Ulmer technique (Non-patent Reference 12) may be utilized. When introducing a mutation as described above, in view of avoiding a change in the fundamental properties (such as physical properties, function, and immunological activity) of the polypeptide, mutual substitution among homologous amino acids (polar amino acids, non-polar amino acids, hydrophobic amino acids, hydrophilic amino acids, positively-charged amino acids, negatively-charged amino acids and aromatic amino acids or the like) may be readily conceived. Furthermore, these usable polypeptides can be modified to the extent that no significant functional change is involved, for example, by modification of its constituent amino group or carboxyl group and the like, such as by amidation and the like.

The aforementioned polypeptide can be produced by common methods that are known in peptide chemistry. A method described in the References (Non-patent documents 13 and 14), for example, may be used, although the methods are not limited thereto, and known methods can be broadly utilized. Particularly, a peptide synthesis method using an ordinary liquid phase method and solid phase method, such as, for example, the Fmoc method, can be exemplified. Moreover, an amino acid synthesizer that is commercially available can be used for producing the polypeptide. Alternatively, genetic manipulation techniques can be also used for producing the polypeptide. For example, a gene encoding a polypeptide of interest can be used to prepare a recombinant DNA (expression vector) that can express the gene in a host cell, followed by transfection of the recombinant DNA (expression vector) into a suitable host cell such as E. coli to obtain a transformant. Culturing the transformant and collecting the polypeptide of interest from the obtained culture product achieves the production of the polypeptide.

The inhibition of the binding of NOD2 to procaspase-1 can be also carried out by using an antibody that recognizes NOD2 or procaspase-1 and inhibits the binding of NOD2 to procaspase-1. Such an antibody can be produced by known methods for preparing an antibody, in which NOD2 or procaspase-1 itself, or a fragment thereof, preferably a polypeptide comprising the amino acid sequence of a site where NOD2 to procaspase-1 is used as an antigen.

A compound that inhibits the binding of NOD2 to procaspase-1 can be obtained by constructing an identification method of the compound by utilizing a known pharmaceutical screening system and using it. For example, a compound that inhibits the binding of NOD2 to procaspase-1 can be identified by selecting the conditions that allow for the binding of NOD2 to procaspase-1, contacting a compound to be tested (hereinafter, referred to a test compound) with NOD2 and/or procaspase-1 under the conditions, employing a system that uses a signal and/or a marker capable of detecting the binding of NOD2 to procaspase-1, and then detecting the presence, the absence, or the change of the signal and/or the marker. For example, in the case that the signal generated by the binding of NOD2 to procaspase-1 or the marker of the binding shows a change, such as reduction or disappearance, when contacting a test compound with NOD2 and/or procaspase-1, it can be determined that the test compound inhibits the binding of NOD2 to procaspase-1. In such an identification method, a test compound may be previously contacted with NOD2 and/or procaspase-1, and then the binding reaction of NOD2 to procaspase-1 may be conducted. Alternatively, a test compound may be allowed to co-exist in the binding reaction of these proteins. The conditions that allow for the interaction of a test compound with NOD2 and/or procaspase-1 may be a condition in vitro or in vivo. For example, a cell in which NOD2 is co-expressed with procaspase-1 may be used. Such a cell can be obtained by transfecting a cell using a suitable vector containing a polynucleotide encoding NOD2 and a suitable vector containing a polynucleotide encoding procaspase-1 by means of conventional genetic manipulation techniques. As used herein, the term "signal" refers to a substance that can be detected itself directly based on its physical properties or chemical properties. The term "marker" refers to a substance that can be indirectly detected based on its physical properties or biological properties as an index. A signal can be exemplified by known markers, such as luciferase, a radioactive isotope, and the like. A marker can be exemplified by known ones, such as a reporter gene, for example chloramphenicol acetyl transferase gene and the like, and epitope tags for detection, for example, 6 x His-tag and the like. A method for detecting these signals or markers is well known to those skilled in the art. The binding of NOD2 to procaspase-1 can be easily detected by detecting the presence or absence of these proteins and/or by measuring the change of the amount thereof. The detection of these proteins and measuring the amount of these proteins can be carried out by a well known method for detecting a protein or peptide, for example, the Western blotting method and the like.

More particularly, a compound that inhibits the binding ofNOD2 to procaspase-1 can be obtained by, for example, evaluating a test compound in an in vitro binding assay system generally known in the art, which comprises immobilizing either of NOD2 or procaspase-1 onto a solid-phase, conducting the binding reaction using the other one that is labeled with the signal, and measuring the labeled signal quantitatively.

Alternatively, a compound that inhibits the binding of NOD2 to procaspase-1 in vivo can be obtained by using an assay system shown in Example 2, that uses a cell co-expressing NOD2 gene with procaspase-1 gene to conduct a binding reaction in the cell, making a test compound act on the assay system, and then detecting the binding of NOD2 to procaspase-1 by a well known method, such as immunoprecipitation and Western blotting.

Moreover, a well known two-hybrid method can also be used. For example, it can be achieved by using a yeast, a eukaryotic cell, or the like into which a plasmid for expressing a fusion protein of NOD2 and DNA binding protein, a plasmid for expressing a fusion protein of procaspase-1 and transcription activating protein and a plasmid containing a reporter gene, such as lacZ or the like, that is linked to a suitable promoter gene are introduced, and comparing an amount of expression of the reporter gene under co-existence of a test compound to an amount of expression of the reporter gene in the absence of the test compound. In the case that the amount of expression of the reporter gene under co-existence of the test compound was reduced, compared to the amount of expression of the reporter gene in the absence of a test compound, it can be determined that the test compound has an activity for inhibiting the binding of NOD2 to procaspase-1.

A compound that inhibits the binding of NOD2 to procaspase-1 can be identified by using a surface plasmon resonance sensor, such as BIACORE system or the like, Scintillation proximity assay (SPA), or a method that employing fluorescence resonance energy transfer (FRET).

Further, a compound that inhibits the oligomerization of procaspase-1 caused by NOD2 can be identified by selecting the conditions that allow for the oligomerization of procaspase-1 by NOD2, contacting a test compound with NOD2 and/or procaspase-1 under the conditions, employing a system that uses a signal and/or a marker capable of detecting the oligomerization of procaspase-1 caused by NOD2, and then detecting the presence, the absence, or the change of the signal and/or the marker. For example, a compound that inhibits the oligomerization of procaspase-1 caused by NOD2 can be identified by using an assay system, such as shown in Example 3, which uses a cell co-expressing NOD2 gene with procaspase-1 gene to determine the oligomerization of caspase-1 in the cell, as shown in Example 3.

Further, a compound that inhibits the activation of procaspase-1 induced by NOD2 can be identified by selecting the conditions that allow for the activation of procaspase-1 by NOD2, contacting a test compound with NOD2 and/or procaspase-1 under the conditions, employing a system that uses a signal and/or a marker capable of detecting the activation of procaspase-1 induced by NOD2, and then detecting the presence, the absence, or the change of the signal and/or the marker. For example, a compound that inhibits the activation of procaspase-1 induced by NOD2 can be identified by selecting a compound capable of reducing or eliminating the amount of IL-1β secreted outside the cell that stably expresses IL-1β and co-expresses NOD2 gene with procaspase-1 gene as shown in Example 4. That compound can be used as a compound capable of inhibiting the production of caspase-1, because of the reason that the production of caspase-1 resulting from the autocleavage of procaspase-1 can be inhibited by inhibiting the activation of procaspase-1 induced by NOD2.

NOD2 and procaspase-1 can be the following: cells in which these are expressed by means of genetic manipulation techniques, the products of cell-free synthesis systems, the chemical synthesis products, or those prepared from such cells or biological samples, as well as those further purified from them. Further, these can be labeled by ligating a different type of protein or polypeptide, for example, β-galactosidase, an immunoglobulin Fc fragment such as IgG, or a tag peptide such as His-tag, Myc-tag, HA-tag, FLAG-tag, or Xpress-tag thereto at the N-terminus or the C-terminus, directly or indirectly via a linker peptide and the like, by means of, for example, genetic engineering techniques, as long as it has no influence upon the binding of NOD2 to procaspase-1, and upon the function of these proteins, as well as upon the feature of caspase-1 that is produced from procaspase-1 through its activation.

In the identification of a compound that inhibits the binding ofNOD2 to procaspase-1 or the oligomerization of procaspase-1 caused by NOD2, it can sometimes be difficult to detect the binding or the oligomerization due to the autocleavage of procaspase-1 following the oligomerization of procaspase-1 caused by the binding. In such a case, in order to easily detect the binding or the oligomerization, a procaspase-1 variant, which can bind to NOD2 to allow oligomerization but does not allow autocleavage, for example, a variant in which cysteine at position 285 in the amino acid sequence was substituted with alanine, may be used.

Examples of a test compound include a compound derived from a chemical library or natural products, as well as a compound obtained by drug design based on the primary structure or tertiary structure of NOD2 or procaspase-1. Alternatively, a compound obtained by drug design based on the structure of a polypeptide that comprises an amino acid sequence of a binding site of NOD2 to procaspase-1 is also suitable as a test compound.

A compound obtained by the identification method described above can be used as an agent for inhibiting the oligomerization of procaspase-1, an agent for inhibiting the activation of procaspase-1, and an agent for inhibiting the production of caspase-1. The compound can be prepared as a pharmaceutical composition by taking into consideration the balance between biological usefulness and toxicity. In preparation of the pharmaceutical composition, these compounds and inhibitors can be used alone or in combination.

The compounds and the inhibiting agents described above can further be used as an agent for preventing and/or treating a disease attributable to the action or the increase of caspase-1, and in a method of preventing and/or treating the disease. As such a disease, for example, sepsis, inflammatory bowel disease, Crohn's disease and rheumatic disorder may be mentioned.

Sepsis is an excessive reaction of the body induced by an inflammatory cytokine which is released by a stimuli such as bacteria and toxins (e.g., endotoxins and exotoxins), namely a systemic inflammatory response syndrome attributable to infection. It is reported that the resistance against endotoxin shock is increased in a caspase-1 gene deficient mouse (Non-patent Document 15). Further, it is reported that the production of IL-1β and IL-18, which are induced by LPS, is almost completely inhibited in a macrophage and monocyte prepared from a caspase-1 gene deficient mouse (Non-patent Document Nos. 4, 15 and 16). From these facts, it can be considered that an endotoxin shock which is caused by the increase of the production of IL-1β and IL-18 secreted from macrophages and monocytes can be inhibited by inhibiting the activity of caspase-1. Meanwhile, the expression of NOD2 gene is observed in monocytes and macrophages (Non-patent Document Nos. 9 and 10). Therefore, the inventors of the present invention believe that prevention and/or treatment of sepsis become possible by inhibiting the binding of NOD2 to procaspase-1, thereby to inhibit the oligomerization of procaspase-1, the activation of procaspase-1, and the production of caspase-1.

Inflammatory bowel disease is an inflammatory disease in the colon and small intestine which comes about by various intractable diseases, such as Crohn's disease and often progresses chronically. It has been reported that colitis is induced in a normal mouse exposed to chronic administration of dextran sulfate (hereinafter, abbreviated as DSS), while it is not induced in a caspase-1 gene deficient mouse exposed to chronic administration of DSS, and that the amount of secretion of IL-1β and IL-18 in colon tissue culture prepared from a caspase-1 gene deficient mouse exposed to DSS-administration is significantly reduced in comparison to that of a normal mouse exposed to DSS-administration (Non-patent Document 17). These results indicate that the increase of caspase-1 activity followed by an increase of secretion of IL-1β and IL-18 can be important in the onset and progress of colitis. Meanwhile, the expression of NOD2 gene is observed in intestinal epithelium cells and macrophages (Non-patent Document 10). Therefore, the inventors of the present invention believe that prevention and/or treatment of inflammatory bowel disease are possible by inhibiting the binding of NOD2 to procaspase-1, to thereby inhibit the oligomerization of procaspase-1, the activation of procaspase-1, and the production of caspase-1.

Rheumatic disorder is reportedly progressed by a cytokine secreted from macrophages present in synovial membrane of joints, and IL-1β and IL-18 also contribute to the progress of rheumatic disorder (Non-patent Document 18). Further, it is reported that administration of a caspase-1 inhibitor to a collagen-induced arthritis model mouse suppresses the inflammation (Non-patent Document 19). These facts indicate that the increase of caspase-1 activity in synovial membrane macrophages followed by an increase of secretion of IL-1β and IL-18 can contribute to the progress of rheumatic disorder. In fact, as a remedy for rheumatic disorder, a caspase-1 inhibitor (Pralnacasan, Vertex Pharmaceutical Inc.) is now studied in the second phase clinical trial. There is no report demonstrating that NOD2 expresses in macrophages present in synovial membrane. However, some reports show that it expresses in macrophages present in the colon (Non-patent Document 10). Therefore, the inventors of the present invention believe that prevention and/or treatment of rheumatic disorder become possible by inhibiting the binding of NOD2 to procaspase-1 to thereby inhibit the oligomerization of procaspase-1, the activation of procaspase-1, and the production of caspase-1.

As an agent for preventing and/or treating a disease according to the present invention, a preventing and/or treating agent can be used that has one or more of the following properties: inhibition of the oligomerization of procaspase-1 by inhibiting the binding of NOD2 to procaspase-1, inhibition of the activation of procaspase-1 by inhibiting the binding of NOD2 to procaspase-1, and/or inhibition of the production of caspase-1 by inhibiting the binding of NOD2 to procaspase-1. A preventing and/or treating agent that inhibits the oligomerization of procaspase-1 by inhibiting the binding of NOD2 to procaspase-1 is preferably used. As such a disease, inflammatory diseases, preferably, sepsis, inflammatory bowel disease, Crohn's disease and rheumatic disorder may be exemplified. Examples of a preventing and/or treating agent include an agent for preventing and/or treating an inflammatory disease comprising an effective amount of at least one selected from an agent for inhibiting procaspase-1 from oligomerization, an agent for inhibiting the activation of procaspase-1, or an agent for inhibiting the production of caspase-1 described above as an effective ingredient may be exemplified.

The agent for preventing and/or treating a disease according to the present invention can be a pharmaceutical agent prepared by using one or more of the aforementioned compounds, the aforementioned agents for inhibiting the oligomerization of procaspase-1, the aforementioned agents for inhibiting the activation of procaspase-1, and/or the aforementioned agents for inhibiting the production of caspase-1, so as to contain the effective amount thereof. Generally, the pharmaceutical agent is preferably prepared as a pharmaceutical composition that includes one or more kinds of a pharmaceutical carrier.

An effective amount of the ingredient contained in the pharmaceutical preparation can be suitably selected from a wide range. In general, a suitable amount may fall within a range of approximately 0.00001 to 70 wt%, preferably approximately 0.0001 to 5 wt%, based on the weight of the pharmaceutical preparation.

A pharmaceutical carrier may be diluents and excipients, which are generally used in accordance with the form of use of the formulation, such as a filler, an extender, a binder, a wetting agent, a disintegrator, a surfactant, and/or a lubricant. These can be suitably selected and used in accordance with the form of administration of the preparation used.

The pharmaceutical carrier may be, for example, water, a pharmaceutically acceptable organic solvent, collagen, polyvinyl alcohol, polyvinylpyrrolidone, carboxyvinyl polymer, sodium alginate, soluble dextran, sodium carboxymethyl starch, pectin, xanthan gum, acacia gum, casein, gelatin, agar, glycerin, propylene glycol, polyethylene glycol, vaseline, paraffin, stearyl alcohol, stearic acid, human serum albumin, mannitol, sorbitol and lactose. One or a combination of two or more kinds of these carriers may be suitably selected and used in accordance with the dosage form of the pharmaceutical composition of the present invention.

As desired, various components used in conventional protein preparations can be used herein, such as a stabilizer, a bacteriocide, a buffer agent, an isotonizing agent, a chelating agent, a pH adjuster, or surfactant, and may be suitably contained in the pharmaceutical composition.

As a stabilizer, the following may be used: human serum albumin, common L-amino acids, sugars and cellulose derivatives. These can be used independently or in combination with a surfactant and the like. Use of these in such a combination may give increased stability of an effective ingredient. An L-amino acid is not particularly limited, and may be any one of glycine, cysteine, glutamic acid and the like. A sugar is not particularly limited, and may be any one of the monosaccharides (such as glucose, mannose, galactose and fructose), sugar alcohols (such as mannitol, inositol and xylitol), disaccharides (such as sucrose, maltose and lactose), polysaccharides (dextran, hydroxypropylstarch, chondroitin sulfate and hyaluronic acid), derivatives thereof, and so on. A cellulose derivative is not particularly limited, and may be any one of methylcellulose, ethylcellulose, hydroxyethylcellulose, hydroxypropylcellulose, hydroxypropylmethylcellulose, sodium carboxymethylcellulose, and the like. A surfactant is not particularly limited, and can be both an ionic surfactant and a non-ionic surfactant. As a surfactant, the following may be used: polyoxyethyleneglycol sorbitan alkyl ester system, polyoxyethylene alkyl ether system, sorbitan monoacyl ester system, or a fatty acid glyceride system.

As a buffer agent, the following may be used: boric acid, phosphoric acid, acetic acid citric acid, ε-aminocaproic acid, glutamic acid, and/or a salt thereof. For example, an alkali metal salt and/or an alkaline earth metal salt, such as sodium salt, kalium salt, calcium salt and magnesium salt) .

As an isotonizing agent, the following may be used: sodium chloride, kalium chloride, sugars or glycerin.

As a chelating agent, sodium edentate and citric acid may be used.

The pharmaceutical agents and pharmaceutical compositions according to the present invention can be used as a solution preparation. Alternatively, they can be freeze-dried so as to be in a good state for preservation, and can be used by dissolving them in water, a buffered solution containing saline and the like, and so on, and then adjusting to a suitable concentration at the time of using.

Suitable dosage ranges of the pharmaceutical composition are not particularly limited, and can be determined according to the following: effectiveness of the ingredients contained therein; the administration form; the route of administration; the type of disease; the characteristics of the subject (e.g., body weight, age, symptomatic conditions and whether subject is taking other pharmaceutical agents) and the judgment of the physician in charge. In general, a suitable dosage may fall, for example, within a range of about 0.01 µg to 100 mg per 1 kg of the body weight of the subject, and preferably within a range of about 0.1 µg to 1 mg per 1 kg. However, the dosage may be altered using conventional experiments for optimization of a dosage that are well known in the art. The aforementioned dosage can be divided for administration once to several times a day. Alternatively, periodic administration once every few days or few weeks can be employed.

When administering the pharmaceutical compositions of the present invention, the pharmaceutical composition may be used alone or may be used together with other compounds or pharmaceutical agents useful for preventing and/or treating the target disorder.

In terms of a route of administration, it may be either systemic administration or local administration. The route of administration that is appropriate for a particular disease, symptomatic conditions, or other factors should be selected. For example, parenteral administration including normal intravenous injection, intraarterial administration, subcutaneous administration, intracutaneous administration, and intramuscular administration can be employed. Oral administration can be also employed. Further, transmucosal administration or dermal administration can be employed.

In terms of an administration form, various forms can be selected in accordance with a treatment purpose. For example, a solid formulation may be used, such as a tablet, a pill, powder, powdered drug, fine granule, granule or a capsule, or a liquid formulation can be used, such as an aqueous formulation, ethanol formulation, suspension, fat emulsion, liposome formulation, clathrate such as cyclodextrin, syrup or an elixir. These can be further classified, according to the administration route, into an oral formulation, parenteral formulation (drip injection formulation or injection formulation), nasal formulation, inhalant formulation, transvaginal formulation, suppositorial formulation, sublingual agents, eye drop formulation, ear drop formulation, ointment formulation, cream formulation, transdermal absorption formulation, transmucosal absorption formulation and the like, which can be respectively blended, formed and prepared according to conventional methods.

The present invention further provides a reagent kit comprising at least one selected from NOD2, a polynucleotide encoding NOD2, a vector comprising the polynucleotide or a transformant comprising the vector; and at least one selected from procaspase-1, a polynucleotide encoding procaspase-1, a vector comprising the polynucleotide or a transformant comprising the vector. The reagent kit can be used, for example, in the identification method for the present invention.

The polynucleotide can be prepared from, for example, a human cDNA library by using genetic manipulation techniques that are well known per se. The vector comprising the polynucleotide and the transformant comprising the vector can be obtained by introducing the polynucleotide of interest into an appropriate DNA expression vector, for example, a vector derived from a bacterial plasmid by using genetic manipulation techniques that are well known per se, or transfecting an appropriate cell with the obtained vector by using a well known method.

The aforementioned reagent kit may include one or more chemicals required for use, such as a signal and/or a marker used for detecting the binding of NOD2 to procaspase-1, buffer solutions, and/or salts. The reagent kit may also include one or more chemicals, such as stabilizers and/or antiseptic agents. At the time of preparation, methods for preparation may be introduced in accordance with the respective chemicals to be used.

Hereinafter, the present invention may be explained more particularly with the following examples; however, the present invention is not limited to the following examples.

### Example 1

### (In-silico Search for Proteins Having a Function to Interact with Procaspase-1)

The prediction of proteins that have a function to interact with procaspase-1 was conducted according to the method described in the Patent Document 1. Particularly, the amino acid sequence of procaspase-1 was decomposed into oligopeptides having a pre-determined length in order to search in a database for proteins having the amino acid sequence of each of the oligopeptides, or having homologous amino acid sequences to these amino acid sequences. Then, local alignment was conducted between the proteins obtained and procaspase-1 to identify proteins having a high local alignment score that might be capable of interacting with procaspase-1.

From the results of analysis, NOD2 was identified as a protein being predicted to have a function to interact with procaspase-1.

### Example 2

### (Binding Analysis between NOD2 and Procaspase-1)

The binding of NOD2 to procaspase-1 was examined by using an in vivo binding assay.

### <Materials and Preparation Thereof>

In this Example, a variant (C285A) of procaspase-1, in which cysteine at position 285 in the amino acid sequence of procaspase- 1 was substituted with alanine, was used for procaspase-1. The procaspase-1 (C285A) was not affected by autocleavage due to this one amino acid substitution. The open reading frame (hereinafter, abbreviated as ORF) of procaspase-1 (C285A) was inserted into pCMV-Tag2 (STRATAGENE) to obtain a plasmid for expression of N-terminal FLAG-tagged procaspase-1 (C285A) (FLAG-procaspase-1 (C285A)). Further, the ORF of NOD2 was inserted into pcDNA3.1/myc-His (INVITROGEN) to obtain a plasmid for expression of C-terminal myc-His-tagged NOD2 (NOD2-myc-His).

### <Method>

HEK293T cell was transfected with a FLAG-procaspase-1 (C285A) expression plasmid alone, an NOD2-myc-His expression plasmid alone, or both in combination using FuGene6 (Roche). Each 2.5 µg of expression plasmid was used respectively. Further, the total amount of the DNA was adjusted to be 5 µg with pCMV-Tag2 or pcDNA3.1 (-)/myc-His. After incubating for 48 hours, a cell lysate was prepared with 0.5 ml of lysis buffer 1 (50 mM Tris-HCl (pH7.6) /150 mM NaCl/0.1% Nonidet P-40 (NP-40)). Each cell lysate was added with a mouse IgG-conjugated agarose (Sigma) or a rabbit IgG-conjugated agarose (Sigma) and mixed for an hour at 4°C by inversion, and then the supernatant was collected. An anti-FLAG M2 antibody-conjugated agarose (Santa Cruz) or an anti-myc antibody-conjugated agarose (Santa Cruz) was added to each supernatant and mixed overnight at 4°C by inversion, and then the supernatant was removed to collect a binding fraction. After the binding fraction was subjected to SDS-PAGE for separation, each protein was detected by Western blotting using an anti-myc antibody (Santa Cruz) and an anti-FLAG M2 antibody. Further, each cell lysate was subjected for detection of each protein by Western blotting.

### <Results>

As shown in Figure 1-A, the expression of the objective protein was observed in the corresponding cell lysate. Further, as shown in the left panel in Figure 1-B, a band of NOD2-myc-His was detected in the cell lysate prepared from the cell co-expressing NOD2-myc-His with FLAG-procaspase-1 (C285A) by immunoprecipitation using the anti-FLAG M2 antibody. The intensity of the band was stronger than that detected in the cell lysate prepared from the cell expressing NOD2-myc-His alone by immunoprecipitation. Thus, it was suggested that NOD2 was coprecipitated with procaspase-1. Further, as shown in the right panel in Figure 1-B, a band of FLAG-procaspase-1 (C285A) was detected only in a cell lysate co-expressing NOD2-myc-His with FLAG-procaspase-1 (C285A) by immunoprecipitation using the anti-myc antibody. Thus, it was suggested that procaspase-1 was coprecipitated with NOD2. From the results above, the inventors of the present invention concluded that NOD2 binds to procaspase-1.

### Example 3

### (Study for NOD2-Induced Facilitation of Oligomerization of Procaspase-1)

It is postulated that the activation of procaspase-1 to caspase-1 is induced by the oligomerization of procaspase-1 followed by its autocleavage. Therefore, we examined the effect of NOD2 on the oligomerization of procaspase-1.

### <Materials and Preparation Thereof>

### Each Expression Plasmid

The ORF of procaspase-1 (C285A) was inserted into pCMV-Tag3 (STRATAGENE) to obtain a plasmid for expression of N-terminal myc-tagged procaspase-1 (C285A) (Myc-procaspase-1 (C285A)). Further, the ORF of NOD2 was inserted into pCMV-Tag5 (STRATAGENE) to obtain a plasmid for expression of C-terminal myc-tagged NOD2 (NOD2-myc). Further, FLAG-procaspase-1 (C285A), prepared in the same manner as in Example 2, was used.

### <Method>

HEK293T cell was transfected with 0.5 µg of a FLAG-procaspase-1 (C285A) expression plasmid, 0.5 µg of a Myc-procaspase-1 (C285A) expression plasmid and 0 to 1.0 µg of a NOD2-myc expression plasmid using FuGene6 (Roche). The total amount of the DNA was adjusted to be 2 µg with pCMV-Tag5. After incubation for 24 hours, a cell lysate was prepared with 0.25 ml/well of lysis buffer 2 (20 mM Tris-HCl (pH7.5) /150 mM NaCl/2 mM ethylenediamine tetraacetic acid (EDTA) /0.5% NP-40). 0.2 ml of each cell lysate was added with 15µl of an anti-FLAG M2 antibody-conjugated agarose and mixed overnight at 4°C by inversion, and then the supernatant was removed to collect the agarose. After washing two times with 0.5 ml of lysis buffer 2, the agarose was added with 15µl of 2 x SDS sample buffer and subjected to heat treatment for 5 minutes at 100°C. The resultant was separated by SDS-PAGE and subjected to Western blotting using anti-myc antibody to measure the amount of Myc-procaspase-1 (C285A) coprecipitated with FLAG-procaspase-1 by binding thereto and thereby to evaluate the level of oligomerization of procaspase-1. Further, each cell lysate was measured for the amount of each expressed protein by Western blotting.

### <Results>

As shown in Figure 2-A, the amount of band detected with anti-myc antibody in a sample collected from the cell lysate using anti-FLAG M2 antibody increased in a manner dependent on the amount of NOD2-myc expression plasmid. This fact indicates that the amount of Myc-procaspase-1 (C285A) that binds to FLAG-procaspase-1 (C285A) increased in a manner dependent on the amount of NOD2-myc expression plasmid. Further as shown in Figure 2-B, it was revealed that any of the cells transfected with each of the expression plasmids expressed Myc-procaspase-1 (C285A) and FLAG-procaspase-1 (C285A) in almost the same amount. From these facts, it was proven that NOD2 facilitated the oligomerization of procaspase-1.

### Example 4

### (Effects of NOD2 on Caspase- Dependent Cleavage of proIL-1β to IL-1β within Cell and Secretion of IL-1β)

It was examined using a proIL-1β stably expressing cell which was transfected with procaspase-1 expression plasmid and/or NOD2 expression plasmid. Whether the presence of NOD2 induces the activation of procaspase-1 to caspase-1 to thereby cause a change in the cleavage of proIL-1β to IL-1β within the cell and in the amount of IL-1β secreted outside the cell was studied.

### <Materials and Preparation Thereof>

### 1. Preparation of Each Expression Plasmid

The ORF of procaspase-1 was inserted into pCMV-Tag2 to obtain a plasmid for expression of N-terminal FLAG-tagged procaspase-1 (FLAG-procaspase-1). Further, NOD2-myc expression plasmid was prepared in the same manner as in Example 3. The ORF of ProIL-1β was inserted into pcDNA3.1/myc-His to obtain a plasmid for expression of C-terminal myc-His-tagged proIL-1β.

### 2. Preparation of proIL-1β stably expressing cell

HEK293 cell was transfected with proIL-1β expression plasmid using FuGene6 (Roche). Thereafter, clones grown on a medium containing 1mg/ml of geneticin were selected. Among the selected clones, the clone in which expression of proIL-1β was observed was used for experiments (the clone may be referred to as ProIL-1β stably Expressing Cell (HEK293)).

### <Method>

### 1. Effects of NOD2 on Cleavage of proIL-1β into IL-1β within Cell

In a 6-well plate, the IL-1β stably expressing cell (HEK293) was seeded at 5 x 10⁵/well and incubated overnight, and then transfected with 0.5 µg of FLAG-procaspase-1 expression plasmid and/or 0.1 to 2.0 µg ofNOD2-myc expression plasmid using FuGene6 (Roche). The total amount of the DNA was adjusted to be 2.5 µg in pCMV-Tag2 or pCMV-Tag5. After incubating for 24 hours, 0.25 ml/well of lysis buffer 2 was added to the cell to prepare a cell lysate.

40 µl of the cell lysate prepared from each well was added with 10 µl of 5 x SDS sample buffer and subjected to heat treatment for 5 minutes at 100°C. After that, the resultant was separated by SDS-PAGE and subjected to Western blotting using an anti-myc antibody to measure the amounts of proIL-1β, IL-1β and NOD2. Further, Western blotting was conducted using an anti-FLAG antibody to measure the amount of procaspase-1.

### 2. Effects ofNOD2 on Caspase-1 Dependent Secretion of IL-1β

In a 6-well plate, the IL-1β stably expressing cell (HEK293) was seeded at 5 x 10⁵/well and incubated overnight, and then transfected with 0.5 µg of FLAG-procaspase-1 expression plasmid and/or 0.1 to 1.0 µg ofNOD2-myc expression plasmid using FuGene6. The total amount of the DNA was adjusted to be 2.5 µg in pCMV-Tag2 or pCMV-Tag5. After incubating for 24 hours, the cultured supernatant was collected. The collected cultured supernatant of each well was diluted two-fold with DMEM containing 10% fetal bovine serum (FCS) and then subjected to the measurement of the amount of IL-1β contained in the cultured supernatant using human IL-1β ELISA kit (Bio Source) according to the attached protocol.

### <Results>

As shown in Figure 3, it was observed that the amount of IL-1β in the cell increased in a manner dependent on the amount of NOD2-myc expressed in a cell co-expressing FLAG-procaspase-1 expression plasmid and NOD2-myc expression plasmid. Thus, it was revealed that NOD2 facilitated the caspase-1 dependent cleavage of proIL-1β to IL-1β in a cell.

As shown in Figure 4, it was observed that the amount of IL-1β secreted outside the cell increased in a manner dependent on the amount of NOD2-myc expression plasmid in a cell co-expressing FLAG-procaspase-1 expression plasmid and NOD2-myc expression plasmid. Thus, it was revealed that NOD2 facilitated the caspase-1 dependent secretion of IL-1β outside a cell.

### INDUSTRIAL APPLICABILITY

The present invention is applicable for preventing and/or treating severe or intractable disorders including an inflammatory disease such as sepsis, inflammatory bowel disease, Crohn's disease and rheumatic disorder, and thus is quite useful in the pharmaceutical field.

## Claims

1. A method selected from the following group:
(i) a method of inhibiting the oligomerization of procaspase-1, comprising inhibiting the binding of NOD2 to procaspase-1,
(ii) a method of inhibiting the activation of procaspase-1, comprising inhibiting the binding of NOD2 to procaspase-1, and
(iii) a method of inhibiting the production of caspase-1, comprising inhibiting the binding of NOD2 to procaspase-1.

2. A method of preventing and/or treating an inflammatory disease, comprising inhibiting the binding ofNOD2 to procaspase-1.

3. A method of preventing and/or treating an inflammatory disease, comprising using at least one compound that inhibits the binding of NOD2 to procaspase-1.

4. The method of preventing and/or treating an inflammatory disease according to claim 2 or claim 3, wherein the inflammatory disease is sepsis, inflammatory bowel disease, Crohn's disease or rheumatic disorder.

5. A method of identifying a compound that inhibits the binding ofNOD2 to procaspase-1, comprising contacting NOD2 and/or procaspase-1 with a compound under conditions that allow for the binding of NOD2 to procaspase-1, employing a system using a signal and/or marker capable of the detecting the binding of NOD2 to procaspase-1; and detecting the presence or absence and/or change of the signal and/or marker to determine whether the compound inhibits the binding ofNOD2 to procaspase-1.

6. An agent selected from the following group:
(i) an agent for inhibiting the oligomerization of procaspase-1, which inhibits the binding of NOD2 to procaspase-1,
(ii) an agent for inhibiting the activation of procaspase-1, which inhibits the binding of NOD2 to procaspase-1, and
(iii) an agent for inhibiting the production of caspase-1, which inhibits the binding of NOD2 to procaspase-1.

7. An agent selected from the following group:
(i) an agent for inhibiting the oligomerization of procaspase-1, comprising at least one compound that inhibits the binding ofNOD2 to procaspase-1,
(ii) an agent for inhibiting the activation of procaspase-1, comprising at least one compound that inhibits the binding ofNOD2 to procaspase-1, and
(iii) an agent for inhibiting the production of caspase-1, comprising at least one compound that inhibits the binding ofNOD2 to procaspase-1.

8. An agent for preventing and/or treating an inflammatory disease, which inhibits the binding of NOD2 to procaspase-1.

9. An agent for preventing and/or treating an inflammatory disease, comprising at least one compound that inhibits the binding ofNOD2 to procaspase-1.

10. An agent for preventing and/or treating an inflammatory disease, comprising the agent according to claim 6 or claim 7.

11. The agent for preventing and/or treating an inflammatory disease according to any one of claims 8 to 10, wherein the inflammatory disease is sepsis, inflammatory bowel disease, Crohn's disease or rheumatic disorder.

12. An agent for preventing and/or treating an inflammatory disease, which inhibits the oligomerization of procaspase-1 by inhibiting the binding ofNOD2 to procaspase-1.

13. The agent for preventing and/or treating an inflammatory disease according to claim 12, wherein the inflammatory disease is sepsis, inflammatory bowel disease, Crohn's disease or rheumatic disorder.

14. A reagent kit for use in the method according to claim 5, comprising at least one selected from NOD2, a polynucleotide encoding NOD2, a vector comprising the polynucleotide and a transformant comprising the vector; and at least one selected from procaspase-1, a polynucleotide encoding procaspase-1, a vector comprising the polynucleotide and a transformant comprising the vector.
